(19) Europäisches Patentamt
European Patent Office
Office européen des brevets

(11) EP 1 185 489 B1

(12) # EUROPÄISCHE PATENTSCHRIFT

(45) Veröffentlichungstag und Bekanntmachung des Hinweises auf die Patenterteilung:
**10.03.2004 Patentblatt 2004/11**

(21) Anmeldenummer: **00938781.2**

(22) Anmeldetag: **09.06.2000**

(51) Int Cl.[7]: **C07B 63/04**, B01J 19/00

(86) Internationale Anmeldenummer:
**PCT/EP2000/005360**

(87) Internationale Veröffentlichungsnummer:
**WO 2000/076943 (21.12.2000 Gazette 2000/51)**

(54) **VERFAHREN ZUR VERHINDERUNG UNERWÜNSCHTER POLYMERISATION IN EINEM ETHYLENISCH UNGESÄTTIGTE VERBINDUNGEN ENTHALTENDEN STOFFGEMISCH**

METHOD FOR PREVENTING UNDESIRED POLYMERISATION IN A MIXTURE OF SUBSTANCES CONTAINING ETHYLENICALLY UNSATURATED COMPOUNDS

PROCEDE PERMETTANT D'EMPECHER UNE POLYMERISATION NON DESIREE DANS UN MELANGE CONTENANT DES COMPOSES ETHYLENIQUEMENT INSATURES

(84) Benannte Vertragsstaaten:
**AT BE CH CY DE DK ES FI FR GB GR IE IT LI LU MC NL PT SE**

(30) Priorität: **11.06.1999 DE 19926758**

(43) Veröffentlichungstag der Anmeldung:
**13.03.2002 Patentblatt 2002/11**

(73) Patentinhaber: **BASF AKTIENGESELLSCHAFT**
**67056 Ludwigshafen (DE)**

(72) Erfinder:
- **SUTORIS, Heinz, Friedrich**
  **D-67551 Worms (DE)**
- **MITULLA, Konrad**
  **D-67071 Ludwigshafen (DE)**

(74) Vertreter: **Kinzebach, Werner, Dr. et al**
**Ludwigsplatz 4**
**67059 Ludwigshafen (DE)**

(56) Entgegenhaltungen:
**WO-A-96/16921** **GB-A- 992 548**

- **DATABASE WPI Section Ch, Week 8536 Derwent Publications Ltd., London, GB; Class A41, AN 1985-222317 XP002150188 & SU 1 139 722 A (EREV OKHTINSK PLAST), 15. Februar 1985 (1985-02-15) in der Anmeldung erwähnt**
- **DATABASE WPI Section Ch, Week 9112 Derwent Publications Ltd., London, GB; Class A13, AN 1991-085387 XP002150189 & SU 1 558 888 A (CHEM REAGENT PURE), 23. April 1990 (1990-04-23) in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 185 489 B1

## Beschreibung

Die vorliegende Erfindung betrifft ein Verfahren zur Verhinderung unerwünschter Polymerisation in einem ethylenisch ungesättigte Verbindungen enthaltenden Stoffgemisch, insbesondere bei der Gewinnung von ethylenisch ungesättigten Verbindungen aus einem diese enthaltenden Stoffgemisch durch Destillation.

Es ist bekannt, dass viele ungesättigte Verbindungen bei Temperaturerhöhung zu in der Regel radikalisch verlaufender Polymerisation neigen. So müssen beispielsweise vinylaromatische Verbindungen, wie Styrol oder $\alpha$-Methylstyrol, mit geeigneten Verbindungen stabilisiert werden, um eine vorzeitige Polymerisation bei der destillativen Reinigung der großtechnisch erhaltenen Rohprodukte zu verhindern. Üblicherweise werden den zu destillierenden Rohprodukten dabei Stabilisatoren oder Polymerisationsinhibitoren vor oder während dem Reinigungsschritt zugesetzt. Trotz dieser Maßnahme erhält man einen gewissen Anteil an Oligomeren oder Polymeren. Im Einzelfall kann, besonders bei Betriebsstörungen, während der Reinigung oder Destillation eine komplette Polymerisation der vorliegenden Monomeren oder des Monomerengemisches erfolgen. Dies führt durch den umfangreichen Reinigungsaufwand und Produktionsausfall zu Unkosten.

Die rein erhaltenen ethylenisch ungesättigten verbindungen müssen weiterhin während der Lagerung oder Handhabung, z.B. bei der Derivatisierung, vor unerwünschter vorzeitiger Polymerisation geschützt werden.

In den sowjetischen Patentschriften SU-1027150, SU-1558888 und SU-1139722 wird die Stabilisierung von Styrol durch Verwendung von Nitroxyl- oder Bis-Nitroxylverbindungen beschrieben.

Die WO-96/16921 offenbart Mischungen aus vinylaromatischen Verbindungen mit sterisch gehinderten Nitroxylverbindungen, welche durch Sauerstoffspuren aktiviert werden.

Aus der JP Hei 1-165534 sind Piperidyloxyderivate als Polymerisationsinhibitoren für Styrol bekannt.

Die GB 992,548 beschreibt die Polymerisation ungesättigter Verbindungen in einem inerten flüssigen Reaktionsmedium in Gegenwart eines Ziegler-Katalysators. Die Konzentration an freien Elektronen, die am Schwermetall des Ziegler-Katalysators lokalisiert sind, wird periodisch oder kontinuierlich, beispielsweise durch Elektronenspinnresonanz (ESR), gemessen und gegebenenfalls angepasst.

In der US-PS-5254760 und der DE-19622498 sind Mischungen von Nitroxyl- und Nitroverbindungen zur Stabilisierung von vinylaromatischen Verbindungen während der Reinigung oder Destillation beschrieben.

Die DE 19651307 beschreibt Stoffmischungen, die vinylgruppenhaltige Verbindungen, wie Styrol sowie eine vorzeitige Polymerisation inhibierende Mischung aus einer N-Oxyl-Verbindung und einer Eisen-Verbindung enthalten. Die Mischungen sind wirksam gegen vorzeitige Polymerisation während der Reinigung oder Destillation stabilisiert.

Die Nitroxyl-Radikale müssen zur Gewährleistung einer ausreichenden Stabilisierung in einer bestimmten Mindestkonzentration in den ethylenisch ungesättigte Verbindungen enthaltenden Stoffgemischen vorliegen. Durch Abfangreaktionen mit spontan sich bildenden Radikalen wird ein gewisser Anteil der Nitroxyl-Radikale fortlaufend verbraucht. Die Geschwindigkeit des Verbrauchs hängt dabei von äußeren Größen, wie der Temperatur, der Gegenwart von Radikalinitiatoren, des Zutritts von Sauerstoff usw. ab. Diese Einflußgrößen können mitunter unvorhergesehen schwanken. Um auch unter ungünstigen Umständen eine ausreichende Stabilisierung zu gewährleisten, müssen die Nitroxylradikale daher in einem mehr oder weniger großen Überschuß zu den zu stabilisierenden Stoffmischungen gegeben werden. Da es sich bei Nitroxylradikalen um vergleichsweise teure Polymerisationsinhibitoren handelt, stellt die erforderliche Menge an Nitroxylradikalen zur Stabilisierung während der Reinigung und/oder Handhabung einen nicht zu vernachlässigenden Kostenfaktor dar. Es ist daher wünschenswert, den Überschuß bei der Dosierung von Nitroxylradikalen so gering wie möglich zu halten beziehungsweise zu vermeiden.

Der vorliegenden Erfindung liegt daher die Aufgabe zugrunde, ein Verfahren zur Verhinderung unerwünschter Polymerisation in einem ethylenisch ungesättigte Verbindungen enthaltenden Stoffgemisch, insbesondere bei der Gewinnung von ethylenisch ungesättigten Verbindungen aus einem diese enthaltenden Stoffgemisch durch Destillation, anzugeben, bei dem der Stabilisator möglichst effizient genutzt wird.

Es wurde nun gefunden, dass die Konzentration der über wenigstens ein ungepaartes Elektron verfügenden N-Oxyl-Radikale vergleichsweise leicht elektronisch festgestellt werden kann, so dass ein Zusatz an Stabilisator wirksam gesteuert werden kann.

Gegenstand der Erfindung ist demzufolge ein Verfahren zur Verhinderung unerwünschter Polymerisation in einem ethylenisch ungesättigte Verbindungen enthaltenden Stoffgemisch durch Aufrechterhalten einer wirksamen Konzentration eines Stabilisatorsystems, das N-Oxyl-Radikale umfasst, wobei

(i) periodisch oder kontinuierlich ein elektronisches Signal gewonnen wird, das mit der Konzentration der N-Oxyl-Radikale in dem Stoffgemisch korreliert,

(ii) das elektronische Signal mit einem Vorgabewert verglichen wird und

(iii) nach Maßgabe des Vergleichs ein Zusatz des Stabilisatorsystems zum Stoffgemisch gesteuert wird.

Die Erfindung betrifft außerdem ein Verfahren zur Verhinderung unerwünschter Polymerisation bei der Gewinnung von ethylenisch ungesättigten Verbindungen aus einem diese enthaltenden Stoffgemisch durch Destillation, wobei das Verfahren die zuvor genannten Schritte (i), (ii) und (iii) umfasst.

Bei einem bevorzugten Verfahren erfolgt die Destillation in einer Kaskade von mehreren Destillationskolonnen, wobei sich im Sumpf mindestens einer Destillationskolonne eine das Stabilisatorsystem enthaltende Hochsiederfraktion anreichert, wobei ein Teilstrom der Hochsiederfraktion entnommen und dem zulauf einer vorgeschalteten Kolonne beigemischt wird.

Die Konzentration der N-Oxyl-Radikale im zu stabilisierenden Stoffgemisch beträgt vorzugsweise wenigstens 0,1 ppm, insbesondere 1 bis 500 ppm, besonders bevorzugt 5 bis 150 ppm, bezogen auf ethylenisch ungesättigte Verbindung.

Erfindungsgemäß eingesetzte N-Oxyl-Radikale sind durch wenigstens ein ungepaartes Elektron gekennzeichnet. Sie können daher leicht z.B. durch ESR- (Elektronenspinresonanz) bzw. EPR- (elektronenparamagnetische Resonanz) -Spektroskopie nachgewiesen werden. Diese Spektroskopieverfahren beruhen auf der Tatsache, dass das Elektron einen Elektronenspin s=1/2 aufweist. Bringt man ein solches Teilchen in ein homogenes magnetisches Gleichfeld $H_0$, so wird durch das Feld eine Kraft auf das Teilchen ausgeübt, die bestrebt ist, sein magnetisches Moment und damit seinen Spinvektor in die Feldrichtung zu drehen. Unter dem Einfluß dieser Kraft führt das Elektron eine Präzessionsbewegung um die Achse des äußeren Feldes aus, die gewöhnlich Larmor-Präzession genannt wird. Fügt man noch ein zweites hochfrequentes Magnetfeld $H_1$ senkrecht zum Gleichfeld $H_0$ hinzu, so tritt Resonanz auf, wenn die Frequenz des $H_1$-Feldes $\nu$ gleich der Frequenz der Larmor-Präzession $\nu_L$ wird. Bei Einstrahlung einer elektromagnetischen Welle passender Frequenz $\nu$ tritt magnetische Resonanz-Absorption ein, deren Größe gemessen werden kann.

Vorzugsweise wird demzufolge das elektronische Signal gewonnen, indem das Stoffgemisch oder eine Teilmenge davon einem magnetischen Feld ausgesetzt wird und gleichzeitig ein elektromagnetisches Wechselfeld eingestrahlt wird, wobei die durch die N-Oxyl-Radikale hervorgerufene Resonanz detektiert wird.

Üblicherweise weist das magnetische Gleichfeld $H_0$ eine konstante Größe im Bereich von etwa 0,34 Tesla auf. Die Frequenz des hochfrequenten Magnetfeldes $H_1$ wird dabei kontinuierlich verändert, bis Resonanz auftritt. Bei einer Feldstärke von 0,34 Tesla liegt die Absorption der N-Oxyl-Radikale im Mikrowellenbereich ($\nu \cong 10^{10}$ Hz). Da beim erfindungsgemäßen Einsatz von N-Oxyl-Radikalen die Frequenz bekannt ist, bei der eine Resonanz zu erwarten ist, kann auf aufwendige Vorrichtungen zum Variieren der Frequenz des Magnetfelds $H_1$ verzichtet werden. Es kann also mit einer festen Frequenz $\nu_f$ eingestrahlt werden, die im oder nahe beim Resonanzmaximum der verwendeten N-Oxyl-Radikale bei gegebenem magnetischem Gleichfeld $H_0$ liegt. Dies verringert den apparativen Aufwand zur Durchführung des erfindungsgemäßen Verfahrens erheblich.

In einer praktischen Ausführungsform verwendet man z.B. eine Durchflußmesszelle mit integriertem Magnet, Resonator und Mikrowellenbrücke. Die Durchflußmesszelle wird z.B. an einer geeigneten Stelle in einer Rohrleitung angeordnet, durch die das zu stabilisierende Stoffgemisch fließt. Oft ist es erwünscht, eine Probe während der ESR-Messung strömungslos zu halten. In diesem Fall wird die Messzelle mit Vorteil in einer Bypass-Leitung angeordnet, die z.B. über einen T-Verbinder und ein Umlenkventil mit der das Stoffgemisch führenden Rohrleitung verbunden ist. Durch Umschalten des ventils wird das Stoffgemisch kurzfristig durch die Bypass-Leitung geleitet, bis die Messzelle gefüllt ist. Dann wird durch erneutes Umschalten des Ventils die Bypass-Leitung wieder von der Rohrleitung entkoppelt. In der Messzelle wird dann das ESR-Spektrum an der ruhenden Probe gemessen. Nach der Messung wird das Ventil wieder umgeschaltet, wodurch die Messzelle gespült und mit frischem Stoffgemisch gefüllt wird. Die Vorgänge des Spülens, Abkoppelns und des Messens werden periodisch wiederholt. Vorzugsweise erfolgen sie automatisiert. Geeignete EPR/ESR-Spektrometer mit Durchflußmesszellen werden z.B. von der Firma Magnettech GmbH, Berlin, unter der Bezeichnung 'Miniscope' vertrieben.

Das primär detektierte Resonanzsignal kann, vorzugsweise mit einem geeignet programmierten Datenprozessor, z.B. durch Integration, Filterung und/oder andere Operationen in ein zweckmäßigeres elektronisches Signal umgewandelt werden. Das gewonnene elektronische Signal korreliert mit der Konzentration der N-Oxyl-Radikale in dem Stoffgemisch. Es kann sich um ein Gleichstromsignal variabler Spannung oder ein Wechselstromsignal variabler Amplitude und/oder Frequenz handeln. Andere Signalformen sind möglich. Geeignete Signalformen sind dem Fachmann der Meß- und Regeltechnik geläufig. Das elektronische Signal kann dabei proportional zur Konzentration der N-Oxyl-Radikale sein, es kann aber auch nach einer beliebigen anderen mathematischen Funktion mit der Konzentration korreliert sein, die einen Rückschluß auf die tatsächliche Konzentration der N-Oxyl-Radikale erlaubt. Das elektronische Signal wird dann mit einem Vorgabewert verglichen. Dieser Vergleich geschieht vorzugsweise automatisiert durch einen hierzu geeigneten Komparator. Der Vergleich kann z.B. durch Bilden eines Differenzsignals zwischen dem elektronischen Signal und dem Vorgabewert erfolgen. Ein Fachmann auf dem Gebiet der Meß- und Regeltechnik kann ohne weiteres eine geeignete Anordnung und Schaltung zur Durchführung des vergleichs bereitstellen.

Bei dem Vorgabewert kann es sich um einen frei wählbaren, konstanten Wert handeln, der z.B. einer empirisch ermittelten, wirksamen Konzentration an N-Oxyl-Radikalen entspricht. Es ist jedoch auch möglich, dass der Vorgabewert seinerseits von weiteren Meßgrößen abhängig ist. Die weiteren Meßgrößen werden vorzugsweise ebenfalls periodisch oder kontinuierlich gemessen und in Form eines elektronischen Signals bereitgestellt. Zu derartigen weiteren Meßgrößen zählen z.B. die Temperatur des Stoffgemisches, die Umgebungstemperatur, das Redoxpotential der Stoffmischung, die NIR-Transmission oder -Absorption, die Trübung, die Viskosität, die Dichte oder der Brechungsindex des Stoffgemisches. So kann der vorgabewert an alle Fälle angepasst werden, bei denen zur sicheren Verhinderung einer unerwünschten Polymerisation vorübergehend eine höhere Stabilisatormenge erforderlich ist, z.B. bei einer Temperaturerhöhung.

Nach Maßgabe des Vergleichs des elektronischen Signals mit dem vorgabewert erfolgt die Steuerung des Zusatzes des Stabilisatorsystems zum Stoffgemisch. Der Zusatz des Stabilisatorsystems erfolgt vorzugsweise mittels einer automatischen Dosierungsanlage, mit der vorwählbare Mengen z.B. einer nachstehend erörterten Lösung des Stabilisatorsystems in einem geeigneten Lösungsmittel zugesetzt werden können. Geeignet sind Dosierpumpen, Mikrodosierpumpen, Differentialdosierpumpen, z.B. mit Doppelschnecken als Förderelement, usw.

In vielen Fällen ist es vorteilhaft, eine kontinuierliche Dosierung des Stabilisatorsystems zum Stoffgemisch vorzusehen, die nach Maßgabe des Vergleichs moduliert wird.

Wird beim Vergleich festgestellt, dass die dem ermittelten elektronischen Signal entsprechende Konzentration der N-Oxyl-Radikale geringer ist als die dem Vorgabewert entsprechende, so werden Maßnahmen eingeleitet, um die Konzentration der N-Oxyl-Radikale zu erhöhen, z.B. eine Zugabe von Stabilisatorsystem zum Stoffgemisch oder eine Erhöhung der Geschwindigkeit der Zugabe. Wird beim vergleich dagegen festgestellt, dass die dem ermittelten elektronischen Signal entsprechende Konzentration höher ist als die dem Vorgabewert entsprechende, so unterbleibt ein Zusatz von Stabilisatorsystem oder die Geschwindigkeit der Zugabe wird verlangsamt.

Der Zusatz des Stabilisatorsystems wird vorzugsweise so gesteuert, dass die Konzentration der N-Oxyl-Radikale in dem Stoffgemisch wenigstens 0,1 ppm, insbesondere 1 bis 1000 ppm, besonders bevorzugt 1 bis 500 ppm, vorzugsweise 5 bis 150 ppm, beträgt.

Beim erfindungsgemäßen Destillationsverfahren wird das Stabilisatorsystem vorzugsweise in den Destillationssumpf beziehungsweise in wenigstens einen Destillationssumpf einer Anordnung von mehreren Destillationsvorrichtungen, wie Destillationskolonnen, gegeben oder dem Zulauf zu einer Destillationsvorrichtung beigemischt.

Bei den Stoffgemischen im Sinne der Erfindung kann es sich um reine ethylenisch ungesättigte Verbindungen oder beliebige homogene oder heterogene Stoffgemische handeln, die ethylenisch ungesättigte Verbindungen in solcher Konzentration enthalten, dass eine Polymerisation, üblicherweise eine radikalische Polymerisation, stattfinden kann.

Ethylenisch ungesättigte Verbindungen im Rahmen der Erfindung sind insbesondere

- $\alpha,\beta$-ethylenisch ungesättigte $C_3$-$C_6$-Monocarbonsäuren oder $C_4$-$C_6$-Dicarbonsäurer, wie Acrylsäure, Methacrylsäure, Maleinsäure, Fumarsäure und Itakonsäure,
- Ester aus $\alpha,\beta$-ethylenisch ungesättigten $C_3$-$C_6$-Monocarbonsäuren oder $C_4$-$C_6$-Dicarbonsäuren, wie Methyl(meth)acrylat, Ethyl(meth)acrylat, n-Butyl(meth)acrylat, Isobutyl(meth)acrylat, tert-Butrl(meth)acrylat und 2-Ethylhexyl(meth)acrylat,
- vinylaromatische Verbindungen, wie Styrol, $\alpha$-Methylstyrol, o-Chlorstyrol, Vinyltoluole, Divinylbenzol, Nitrostyrol, Styrolsulfonsäure,
- heteroaromatische Vinylverbindungen, wie Vinylpyridin,
- Vinylester von $C_1$-$C_{18}$-Monocarbonsäuren oder Dicarbonsäuren, wie Vinylacetat, Vinylpropionat, Vinyl-n-butyrat, Vinyllaurat und Vinylstearat,
- lineare oder verzweigtkettige 1-Olefine oder cyclische Olefine, wie z.B. Propen, Buten, i-Buten, Penten, Cyclopenten, Hexen, Cyclohexen, Octen, 2,4,4-Trimethyl-1-penten, gegebenenfalls in Mischung mit 2,4,4-Trimethyl-2-penten, $C_8$-$C_{10}$-Olefin, 1-Dodecen, $C_{12}$-$C_{14}$-Olefin, Octadecen, 1-Eicosen, $C_{20}$-$C_{24}$-Olefin,
- Acrylnitril, Methacrylnitril.
- Vinyl- und Allylalkylether mit 1-40 Kohlenstoffatomen im Alkylrest, wobei der Alkylrest noch weitere Substituenten wie eine Hydroxylgruppe, eine Amino- oder Dialkylaminogruppe oder eine beziehungsweise mehrere Alkoxylatgruppen tragen kann wie z.B. Methylvinylether, Ethylvinylether, Propylvinylether, Isobutylvinylether, 2-Ethylhexylvinylether, Vinylcyclohexylether, Vinyl-4-hydroxybutylether, Decylvinylether, Dodecylvinylether, Octadecylvinylether, 2-(Diethylamino)ethylvinylether, 2-(Di-n-butylamino)ethylvinylether, Methyldiglycolvinylether sowie die entsprechenden Alllylether, beziehungsweisae deren Gemische,
- Acrylamide und alkylsubstituierte Acrylamide wie z.B. Acrylamid, Methacrylamid, N-t-Butylacrylamid, N-Methyl(meth)acrylamid,
- Vinylhalogenide und vinylidenhalogenide, wie Vinylchlorid, Vinylidenchlorid, Vinylfluorid, Vinylbromid,
- mehrfach ethylenisch ungesättigte Verbindungen, wie Butadien und Chloropren,
- sulfogruppenhaltige Monomere, wie Allylsulfonsäure, Methallylsulfonsäure, Vinylsulfonsäure, Allyloxybezolsulfonsäure, 2-Acrylamido-2-methylpropansulfonsäure, deren Alkali- oder Ammoniumsalze, Sulfopropylacrylat, Sulfopropylmethacrylat,

- $C_1$-$C_4$-Hydroxyalkylester von ethylenisch ungesättigten $C_3$-$C_6$-Monocarbonsäuren oder $C_4$-$C_6$-Dicarbonsäuren, insbesondere der Acrylsäure, Methacrylsäure oder Maleinsäure, oder die mit 2-50 Mol Ethylenoxid, Propylenoxid, Butylenoxid oder Mischungen davon alkoxylierte Derivate, oder Ester von mit 2-50 Mol Ethylenoxid, Propylenoxid, Butylenoxid oder Mischungen davon alkoxylierten $C_1$-$C_{18}$-Alkoholen mit den genannten Säuren, wie z.B. Hydroxyethyl(meth)acrylat, Hydroxypropyl(meth)acrylat, Butandiol-1,4-monoacrylat, Ethyldiglycolacrylat, Methylpolyglycolacrylat (11 EO), Methacrylsäureester von mit 3, 5, 7, 10 oder 30 Mol Ethylenoxid umgesetzten $C_{13}$/$C_{15}$-Oxoalkoholen beziehungsweise deren Gemische,
- Vinylphosphonsäure, Vinylphosphonsäuredimethylester und andere phosphorhaltige Monomere,
- Alkylaminoalkyl(meth)acrylate oder Alkylaminoalkyl(meth)acrylamide oder deren Quaternisierungsprodukte, wie z.B. 2-(N,N-Dimethylamino)ethyl(meth)äcrylat, 3-(N,N-Dimethylamino)propyl(meth)acrylat, 2-(N,N-Triethylammonium)ethyl(meth)acrylat-chlorid, 2-Dimethylaminoethyl(meth)acrylamid, 3-Dimethylaminopropyl(meth)acrylamid, 3-Trimethylammoniumpropyl(meth)acrylamid-chlorid,
- Allylester von $C_1$-$C_{30}$-Monocarbonsäuren,
- N-Vinylverbindungen, wie N-Vinylformamid, N-Vinyl-N-methylformamid, N-Vinylpyrrolidon, N-vinylimidazol, 1-Vinyl-2-methylimidazol, 1-Vinyl-2-methylimidazolin, N-Vinylcaprolactam, Vinylcarbazol,
- Diallyldimethylammoniumchlorid,
- Acrolein, Methacrolein,
- 1,3-Diketogruppen enthaltende Monomere wie z.B. Acetoacetoxyethyl(meth)acrylat oder Diacetonacrylamid,
- harnstoffgruppenhaltige Monomere, wie Ureidoethyl(meth)acrylat, Acrylamidoglycolsäure, Methacrylamidoglycolatmethylether,
- Silylgruppen enthaltende Monomere, wie z.B. Trimethoxysilylpropyl(meth)acrylat,
- Glycidylgruppen enthaltende Monomere, wie z.B. Glycidyl(meth)acrylat.

Die Erfindung ist insbesondere geeignet zur Verhinderung unerwünschter Polymerisation von vinylaromatischen Verbindungen enthaltenden Stoffgemischen, insbesondere bei deren Destillation. Typische Stoffgemische, die eine oder mehrere der vorgenannten ethylenisch ungesättigten Verbindungen enthalten, sind z.B. die bei der Herstellung der ungesättigten Verbindungen aus geeigneten Vorläuferverbindungen primär erhaltenen Rohgemische, aus denen die reinen ethylenisch ungesättigten Verbindungen durch Destillation oder andere Aufarbeitungsverfahren gewonnen werden. Ein bevorzugtes Beispiel ist Rohstyrol, d.h. ein bei der Herstellung von Styrol aus Ethylbenzol anfallendes Rohgemisch, das neben Styrol und Ethylbenzol untergeordnete Mengen an Toluol, Benzol, Cumol und/oder $\alpha$-Methylstyrol enthält. Daneben enthält Rohstyrol typischerweise bis zu 3 Gew.-%, z.B. 0,5 bis 1,2 Gew.-%, bezogen auf Styrol, Bestandteile mit einem höheren Siedepunkt als Styrol (sog. Höhersieder), wie Stilbene, Styrololigomere und -polymere sowie Diphenylethan und 2-Phenylnaphthalin. Typische Gemische weisen z.B. folgende Zusammensetzung auf: 1% Benzol, 2% Toluol, 40% Ethylbenzol, 56% Styrol und 1% Höhersieder.

Ein weiteres typisches Beispiel sind die bei der Veresterung von Acrylsäure oder Methacrylsäure mit ein- oder mehrwertigen Alkoholen anfallende Veresterungsgemische aus denen das reine Alkyl(meth)acrylat durch Destillation isoliert werden kann.

Ein weiteres typisches Stoffgemisch ist eine Steamcrackerfraktion mit einem hohen Anteil an $\alpha$-Olefinen.

Ein weiterer Anwendungsbereich der Erfindung ist die chemische Umsetzung von ethylenisch ungesättigten Verbindungen in Reaktionen, an denen die C-C-Doppelbindung nicht beteiligt ist, z.B. die Quaternisierung Aminogruppen-enthaltender ethylenisch ungesättigter Verbindungen.

Im Rahmen der Erfindung gelangt ein Stabilisatorsystem zur Anwendung, das N-Oxyl-Radikale umfasst. Es handelt sich bei den N-Oxyl-Radikalen um stabile freie Radikale, die bisweilen auch als beständige oder persistente Radikale bezeichnet werden. Sie besitzen ein oder mehrere ungepaarte Elektronen. Sie sind in der Regel als Reinsubstanz herstellbar und über Jahre unzersetzt lagerstabil. Sie sind selbst nicht in der Lage, eine radikalisch initiierte Polymerisation auszulösen. Sie fangen bereitwillig organische Radikale ab, die sich spontan z.B. bei der Destillation ethylenisch ungesättigter Verbindungen bilden. In der Regel sind die N-Oxyl-Radikale sterisch gehindert, d.h. sie leiten sich von einem sekundären Amin ab, dessen Wasserstoffatome in $\alpha$-Position zum Stickstoffatom, das die Oxylgruppe trägt, sämtlich z.B. durch Alkylgruppen substituiert sind.

Das Stabilisatorsystem kann neben den N-Oxyl-Radikalen weitere Komponenten, wie die nachstehend erläuterten Polymerisationsverzögerer oder Aktivatoren, enthalten.

Geeignete N-Oxyle weisen z.B. die folgenden Strukturen auf

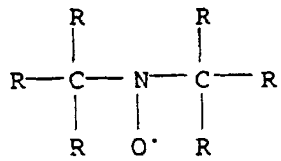

oder

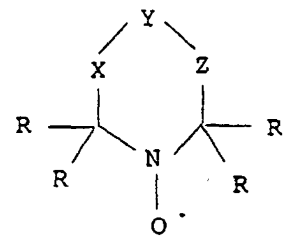

worin R für gleiche oder verschiedene Alkyl-, Cycloalkyl-, Aralkyl-oder Arylreste mit bis zu 24 C-Atomen steht, wobei geminale R-Reste auch paarweise zu einem Ringsystem verbunden sein können, und X, Y und Z unabhängig voneinander für $CR'_2$, CR'OH, CR'(COOH), O, S, CO oder eine chemische Bindung stehen, mit der Maßgabe, dass maximal ein Rest X, Y oder Z für O oder S und maximal ein Rest X, Y oder Z für eine chemische Bindung steht. R' steht für Wasserstoff oder einen Alkyl-, Cycloalkyl-, Aralkyl- oder Arylrest mit bis zu 24 C-Atomen. Beispielsweise steht R für einen $C_1$-$C_{20}$-, insbesondere $C_1$-$C_8$-Alkylrest, einen $C_5$- oder $C_6$-Cycloalkylrest, einen Benzylrest oder einen Phenylrest. X-Y-Z ist beispielsweise $-(CH_2)_2-$ oder $-(CH_2)_3-$, $-CH_2-CH(OH)-CH_2-$, $-CH_2-CO-O$oder $-CH_2-O-$.

Weiterhin kommen auch N-Oxylverbindungen mit aromatischen Substituenten wie die folgenden Strukturen in Betracht

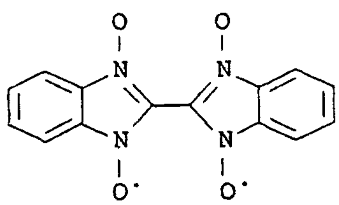

,

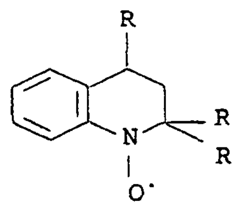

oder

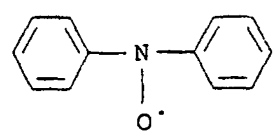

wobei die aromatischen Ringe jeweils noch 1 bis 3 inerte Substituenten tragen können, wie z.B. $C_1$-$C_4$-Alkyl, $C_1$-$C_4$-Alkoxy, Ester, Amid oder Cyano.

Vorzugsweise werden N-Oxyl-Radikale eingesetzt, die sich von cyclischen Aminen ableiten, z.B. von Piperidin- oder Pyrrolidinverbindungen, die im Ring ein weiteres Heteroatom wie Stickstoff, Sauerstoff oder Schwefel enthalten können, wobei dieses Heteroatom nicht in Nachbarstellung zum Aminstickstoff steht. Die sterische Hinderung ist durch Substituenten in beiden Nachbarstellungen zum Aminstickstoff gegeben, wobei als Substituenten Kohlenwasserstoffreste in Betracht kommen, die alle 4 Wasserstoffatome der $\alpha$-$CH_2$-Gruppen ersetzen. Beispielsweise seien als Substituenten Phenyl, $C_3$-$C_6$-Cycloalkyl, Benzyl und insbesondere $C_1$-$C_6$-Alkylreste genannt, wobei die an demselben $\alpha$-C-Atom gebundenen Alkylreste auch untereinander zu einem 5- oder 6-Ring verbunden sein können. Vorzugsweise werden als N-Oxyle sterisch gehinderter Amine Derivate des 2,2,6,6-Tetraalkylpiperidins eingesetzt.

Bevorzugte N-Oxyl-Verbindungen sind solche der allgemeinen Formel (II) oder (II')

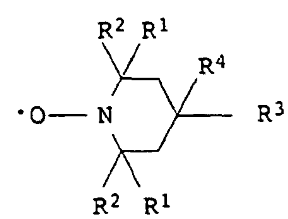

(II),

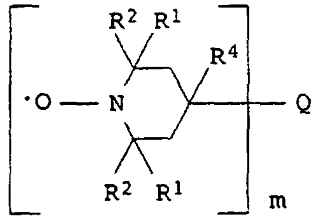

(II')

wobei

$R^1$ und $R^2$ unabhängig voneinander jeweils $C_1$-$C_4$-Alkyl, Phenyl oder $R^1$ und $R^2$ gemeinsam mit dem C-Atom, an das sie gebunden sind, einen 5- oder 6-gliedrigen, gegebenenfalls substituierten, gesättigten Kohlenwasserstoffring, der 1 oder 2 Heteroatome, ausgewählt unter O, S oder N, sowie 1 oder 2 Ketogruppen enthalten kann,

$R^3$ Wasserstoff, Hydroxy, Amino, $SO_3H$, $SO_3M$, $PO_3H_2$, $PO_3HM$, $PO_3M_2$, siliciumorganische Reste oder einen einwertigen über Kohlenstoff, Sauerstoff oder Stickstoff gebundenen organischen Rest mit vorzugsweise 1 bis 36 Atomen, wobei M für ein Alkalimetall, vorzugsweise Li, Na und K, steht,

$R^4$ Wasserstoff, $C_1$-$C_{12}$-Alkyl, $C_1$-$C_{12}$-Alkoxy

oder $R^3$ und $R^4$ gemeinsam Sauerstoff
oder $R^3$ und $R^4$ gemeinsam mit dem C-Atom, an das sie gebunden sind, einen 5- oder 6-gliedrigen, gegebenenfalls substituierten, gesättigten Ring bilden, der 1 oder 2 Heteroatome, ausgewählt unter O, S oder N, sowie 1 oder 2 Ketogruppen enthalten kann,

Q einen m-wertigen über Kohlenstoff, Sauerstoff oder Stickstoff gebundenen organischen Rest mit vorzugsweise 2 bis 10 000 Atomen, insbesondere 4 bis 2000 Atomen,

m 2 bis 100, vorzugsweise 2 oder 3,

bedeuten.

$R^1$ und $R^2$ können $C_1$-$C_4$-Alkylgruppen, wie Methyl, Ethyl, n-Propyl, iso-Propyl, n-Butyl, iso-Butyl, sec-Butyl oder tert.-Butyl sein oder sie können zusammen eine Tetra- oder Pentamethylengruppe bilden. vorzugsweise sind $R^1$ und $R^2$ Methylgruppen.

Als $R^4$ kommen beispielsweise Wasserstoff, die oben genannten $C_1$-$C_4$-Alkylgruppen sowie Pentyl, sec-Pentyl, tert-Pentyl, Neopentyl, 2,3-Dimethyl-but-2-yl, Hexyl, 2-Methylpentyl, Heptyl, 2-Methylhexyl, 2-Ethylhexyl, Octyl, Isooctyl, 2-Ethylhexyl, Nonyl, 2-Methylnonyl, Isononyl, 2-Methyloctyl, Decyl, Isodecyl, 2-Methylnonyl, Undecyl, Isoundecyl, Dodecyl und Isododecyl in Betracht.

Bevorzugte Reste $R^3$ sind wasserstoff,
$C_1$-$C_{20}$-Alkylgruppen, wie Methyl, Ethyl, n-Propyl, iso-Propyl, n-Butyl, iso-Butyl, Pentyl,
Hydroxy,
$C_2$-$C_{20}$-Alkoxygruppen, wie Methoxy, Ethoxy, Propoxy, und t-Butoxy,

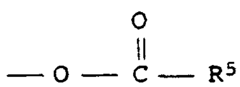 , 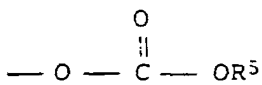 ,

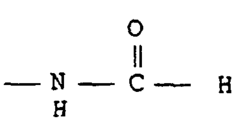 , 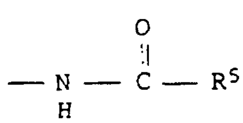 , 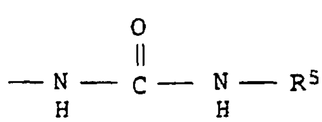 ,

wobei $R^5$ $C_1$-$C_{12}$-Alkyl, $C_6$-$C_{12}$-Aryl oder $C_7$-$C_{14}$-Aralkyl bedeutet,
sowie siliciumorganische Reste der Formel

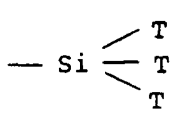

wobei die Gruppen T gleich oder verschieden voneinander sein können und $C_1$-$C_{12}$-Alkyl oder Phenyl bedeuten.

Beispiele solcher siliciumorganischer Reste sind $-Si(CH_3)_3$ und $-Si(C_2H_5)_3$. $R^3$ und $R^4$ können gemeinsam mit dem C-Atom, an das sie gebunden sind, z.B. für

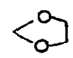

stehen.

Bevorzugte Reste Q sind beispielsweise die folgenden Reste

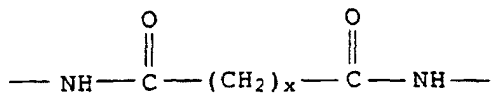
,

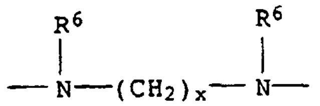
,
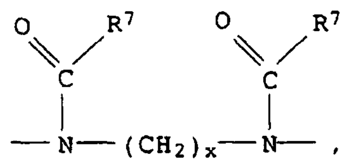
,

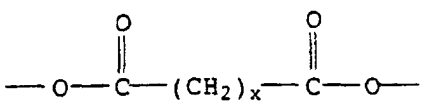
,
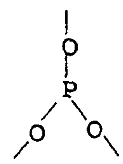
,

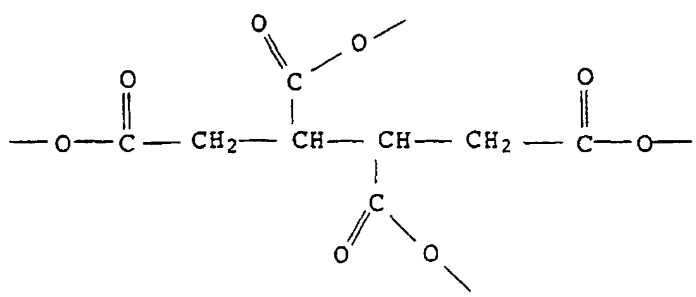

wobei

$R^6$ $C_1$-$C_{12}$-Alkyl,

$R^7$ Wasserstoff oder $C_1$-$C_{18}$-Alkyl,

x 1 bis 12

bedeuten.

Weitere geeignete N-Oxyle sind auch oligomere oder polymere Verbindungen, welche als Polymerhauptkette ein Polysiloxan besitzen und in der Seitenkette mit N-Oxyl-Gruppierungen substituiert sind, welche sich vom 2,2,6,6-Tetraalkylpiperidin ableiten. Als bevorzugte N-Oxylgruppierung wird dabei der 2,2,6,6-Tetramethylpiperidin-N-oxyl-Rest verwendet. Beispiele für solche erfindungsgemäß ebenfalls einzusetzende N-Oxyle finden sich in der WO 69/17002. weiter sind in dieser Druckschrift Beispiele für Synthesen der den N-Oxylen zugrundeliegenden Aminoverbindungen

aufgeführt.

Weitere erfindungsgemäß geeignete N-Oxyl-Radikale sind die in der DE 19651307 als Bestandteil der dort offenbarten Stoffmischung genannten N-Oxyl-Radikale. Auf diese Druckschrift wird vollinhaltlich Bezug genommen.

Bevorzugte Nitroxylverbindungen sind die folgenden:

1-Oxyl-2,2,6,6-tetramethylpiperidin,
1-Oxyl-2,2,6,6-tetramethylpiperidin-4-ol,
1-Oxyl-2,2,6,6-tetramethylpiperidin-4-on,
1-Oxyl-2,2,6,6-tetramethylpiperidin-4-yl-acetat,
1-Oxyl-2,2,6,6-tetramethylpiperidin-4-yl-2-ethylhexanoat,
1-Oxyl-2,2,6,6-tetramethylpiperidin-4-yl-stearat,
1-Oxyl-2,2,6,6-tetramethylpiperidin-4-yl-benzoat,
1-Oxyl-2,2,6,6-tetramethylpiperidin-4-yl-(4-tert-butyl)benzoat,
Bis(1-oxyl-2,2,6,6-tetramethylpiperidin-4-yl)-succinat,
Bis(1-oxyl-2,2,6,6-tetramethylpiperidin-4-yl)-adipat,
Bis(1-oxyl-2,2,6,6-tetramethylpiperidin-4-yl)-sebacat,
Bis(1-oxyl-2,2,6,6-tetramethylpiperidin-4-yl)-n-butylmalonat,
Bis(1-oxyl-2,2,6,6-tetramethylpiperidin-4-yl)-phthalat,
Bis(1-oxyl-2,2,6,6-tetramethylpiperidin-4-yl)-isophthalat,
Bis(1-oxyl-2,2,6,6-tetramethylpiperidin-4-yl)-terephthalat,
Bis(1-oxyl-2,2,6,6-tetramethylpiperidin-4-yl)-hexahydroterephthalat,
N,N'-Bis(1-oxyl-2,2,6,6-tetramethylpiperidin-4-yl)-adipinamid,
N-(1-Oxyl-2,2,6,6-tetramethylpiperidin-4-yl)-caprolactam,
N-(1-oxyl-2,2,6,6-tetramethylpiperidin-4-yl)-dodecylsuccinimid,
2,4,6-Tris-[N-butyl-N-(1-oxyl-2,2,6,6,-tetramethylpiperidin-4-yl]-s-triazin,
N,N'-Bis(1-oxyl-2,2,6,6-tetramethylpiperidin-4-yl)-N,N'-bisformyl-1,6-diaminohexan,
4,4'-Ethylenbis(1-oxyl-2,2,6,6-tetramethylpiperazin-3-on) und Tris-(2,2,6,6-tetramethyl-1-oxyl-piperidin-4-yl)phosphit.

Die Herstellung der erfindungsgemäß verwendeten N-Oxyl-Radikale ist über verschiedene an sich bekannte Syntheseschritte möglich. Eine bevorzugte Herstellungsweise bedient sich der Oxidation eines sekundären Amins, dessen NH-Gruppe oxidativ in die entsprechende N-Oxylgruppe überführt wird. Als Oxidationsmittel kommen Peroxide, wie $H_2O_2$, t-Butylhydroperoxid, Cumolhydroperoxid, Persäuren, wie Metachlorperbenzoesäure, $\alpha$-Chlorperbenzoesäure, Peressigsäure, Paranitroperbenzoesäure, Perbenzoesäure oder Magnesiummonoperoxyphthalat in Betracht. Die Oxidation kann in einem inerten Lösungsmittel, wie $CH_2Cl_2$, Petrolether, Toluol, Xylol oder Benzol, erfolgen.

Die zugrundeliegenden sekundären Amine sind entweder literaturbekannt oder können vom Fachmann der organisch chemischen Synthese durch Abwandlung an sich bekannter Verfahren ohne weiteres hergestellt werden. Die DE 19651307 offenbart die Herstellung verschiedener erfindungsgemäß geeigneter N-Oxyl-Radikale.

Das Stabilisatorsystem kann in Substanz oder in Form einer Lösung in einem Lösungsmittel, wie Wasser, $C_1$-$C_6$-Alkanolen, wie Methanol, Ethanol, Propanol sowie n-, i-, t-Butanol, gegebenenfalls in Mischung mit Wasser, Ketone, wie Aceton, Methylethylketon, Methylpropylketon, Methylbutylketon, Diolen, wie Glycol oder Propylenglycol, sowie deren Alkylmono- und -diether, oligomere oder polymere Ethylenglycole und Propylenglycole sowie deren Alkylether, Diamine, wie Ethylendiamin oder Propylendiamin sowie deren Alkylmono- oder -diiminoether, oligomere oder polymere Ethylendiamine sowie deren Alkyliminoether, zugegeben werden. Vorzugsweise wird jedoch das zu stabilisierende Gemisch als Lösungs- oder Suspendiermittel für das Stabilisatorsystem verwendet. So kann das bei der Dehydrierung von Ethylbenzol anfallende Gemisch, welches überwiegend aus Styrol, Ethylbenzol, Toluol sowie weiteren substituierten Aromaten besteht, zu diesem Zweck eingesetzt werden.

Ein wichtiger Anwendungsfall der Erfindung ist die destillative Isolierung von ethylenisch ungesättigten Verbindungen aus einem entsprechenden Rohgemisch, z.B. die destillative Isolierung von Styrol aus Rohstyrol. Im Folgenden wird die Erfindung anhand der Gewinnung von Styrol aus Rohstyrol veranschaulicht, sie ist jedoch nicht darauf beschränkt. Sämtliche Ausführungen gelten, soweit aus dem Kontext nicht anders ersichtlich, entsprechend für andere ethylenisch ungesättigte Verbindungen und diese enthaltende Stoffgemische.

Eine typische Anordnung zur technischen Destillation von Styrol ist im Kunststoff-Handbuch, Band 4 (Polystyrol), Kap. 2.3.1.4, 30 ff. (München 1996) beschrieben.

wegen der nahe beieinander liegenden Siedepunkte von Styrol und Ethylbenzol (145 bzw. 136°C bei atmosphärischem Druck) und der hohen Anforderungen an die Reinheit des Styrols verlangt dessen Reingewinnung einen hohen Destillationsaufwand. Die Reinigung erfolgt in der Regel durch Destillation in einer Kaskade von mehreren Destillationskolonnen, wobei der Sumpfaustrag einer Destillationskolonne jeweils in die nachgeschaltete Destillationskolonne eingespeist wird. Die Anzahl der aufeinanderfolgenden Kolonnen sei nachfolgend mit n bezeichnet. Die Einspeisung erfolgt vorzugsweise jeweils im Bereich der Kolonnenmitte. In die erste Kolonne wird als Zulauf das styrolhaltige Gemisch eingespeist. Der Parameter n steht für eine positive ganze Zahl ≥ 2 und gibt die Anzahl der Destillationskolonnen in der Kaskade an. Im Allgemeinen ist bevorzugt, dass n für 2 bis 4, z.B. 2 oder 3 steht. In der n-ten Destillationskolonne wird in der Regel Reinstyrol über Kopf abgezogen, während in den der n-ten Kolonne vorgeschalteten Destillationskolonnen die leichter als Styrol siedenden Bestandteile des Rohstyrols über Kopf abgezogen werden. Der Sumpfaustrag der n-ten Kolonne kann zur Isolierung von Restmengen an Styrol und/oder Methylstyrolen einem Konzentrator, z.B. einem Dünnfilmverdampfer oder Flashverdampfer, zugeführt werden. Der dabei gewonnene Leichtsiederanteil kann in einer Aufarbeitungskolonne weiter aufgetrennt werden. Die Anordnung und Verschaltung der einzelnen Destillationskolonnen zur Durchführung des erfindungsgemäßen Verfahrens kann der Fachmann ohne weiteres aufgrund seines fachmännischen Ermessens festlegen. An einer geeigneten Stelle, z.B. in wenigstens einem Kolonnensumpf oder einer Rohrleitung, wird eine Vorrichtung zur Gewinnung des elektronischen Signals vorgesehen.

In die erste Destillationskolonne wird als Zulauf das styrolhaltige Gemisch eingespeist. In einer geeigneten Ausführungsform der Erfindung ist im Sumpf wenigstens einer nachgeschalteten Kolonne oder in der Rohrleitung, über die der Sumpfaustrag in die nächste Kolonne geleitet wird, eine Vorrichtung zur online-ESR-Messung vorgesehen. Das Stabilisatorsystem wird vorzugsweise in wenigstens eine der n-ten Destillationskolonne, z.B. in die erste Destillationskolonne, vorgeschalteten Destillationskolonne gegeben. Das Stabilisatorsystem kann zweckmäßigerweise dem Zulauf einer Destillationskolonne beigemischt werden oder aber in den Sumpf der Kolonne gegeben werden. Die Zugabe des Stabilisatorsystems wird erfindungsgemäß gesteuert, indem das elektronische Signal, das mit der Konzentration der N-Oxyl-Radikale in der Rohstyrol-Kolonne korreliert, mit einem Vorgabewert verglichen wird, der so gewählt ist, dass er einer Konzentration an N-Oxyl-Radikalen von z.B. 1-1000 ppm entspricht.

Bei den N-Oxyl-Radikalen und den fakultativen Komponenten des Stabilisatorsystems handelt es sich um schwerflüchtige Verbindungen. Im Sumpf der n-ten Destillationskolonne reichert sich daher eine Hochsiederfraktion an, die das Stabilisatorsystem enthält.

In einer bevorzugten Ausführungsform wird ein Teilstrom der sich im Sumpf der n-ten Destillationskolonne anreichernden Lösung des Stabilisatorsystems in der Hochsiederfraktion zurückgeführt und zum Zulauf zu wenigstens einer der n-ten Destillationskolonne vorgeschalteten Destillationkolonne gegeben. Der zurückgeführte Strom kann aufgeteilt und an mehreren Punkten zugegeben werden, z.B. zum Zulauf der ersten und zum Zulauf der zweiten Kolonne. Zweckmäßigerweise wird die zurückgeführte Stabilisatorlösung dem Zulauf einer vorgeschalteten Destillationskolonne beigemischt; die zurückgeführte Lösung kann aber auch direkt in den Sumpf einer vorgeschalteten Destillationskolonne gegeben werden.

In der Regel ist es bevorzugt, die dem Sumpf der n-ten Destillationskolonne entnommene Hochsiederfraktion vor der Rückführung bzw. dem Ausleiten aufzukonzentrieren, d.h. von Leichtsiedern zu befreien. Hierzu sind beispielsweise Vorrichtungen, wie ein Dünnfilmverdampfer oder Flächenverdampfer geeignet. Die dabei gewonnene Leichtsiederfraktion kann in einer Aufarbeitungskolonne nochmals in Styrol und α- oder β-Methylstyrol aufgetrennt werden. Nach der Konzentrierung beträgt die Konzentration der N-Oxyl-Radikale in der Hochsiederfraktion im Allgemeinen 0,2 bis 100 g/l.

Die N-Oxyl-Radikale werden vorzugsweise in solcher Menge eingesetzt, dass die Konzentration der N-Oxyl-Radikale im Sumpf jeder Destillationskolonne wenigstens 0,1 ppm, insbesondere 1 bis 500, vorzugsweise 5 bis 150 ppm beträgt. Die Menge im Sumpf einer Destillationskolonne setzt sich aus der gegebenenfalls zurückgeführten und frisch zugegebenen Menge an N-Oxyl-Radikal zusammen. Das erfindungsgemäße Verfahren ist besonders vorteilhaft, wenn ein Teil der Hochsiederfraktion zurückgeführt wird, weil es optimale Ausnutzung der im zurückgeführten Anteil enthaltenen aktiven N-Oxyl-Radikale gestattet. Die Dosierung von frischem Stabilisatorsystem wird so gesteuert, dass lediglich die Differenz zwischen zurückgeführter Menge an N-Oxyl-Radikalen und vorgegebener Wirkkonzentration zugegeben wird. Höhere Überschüsse, die bei bisherigen Verfahren aufgrund der nicht genau bekannten N-Oxyl-Radikalkonzentration aus Sicherheitsgründen unvermeidlich waren, sind beim erfindungsgemäßen Verfahren nicht erforderlich.

Die erfindungsgemäß verwendeten N-Oxyl-Radikale sind wirksame Inhibitoren der Styrolpolymerisation und drängen die Bildung von Styrolpolymeren während der Destillation stark zurück. Im Sumpf der n-ten Destillationskolonne herrscht in der Regel eine höhere Temperatur als in den Sümpfen der vorgeschalteten Kolonnen, da in den vorgeschalteten Kolonnen niedriger als Styrol siedende Fraktionen abdestilliert werden, während in der n-ten Kolonne Styrol über Kopf abgezogen wird. Es wird angenommen, dass im Sumpf der n-ten Destillationskolonne eine teilweise Reaktivierung der N-Oxyl-Radikale stattfindet. Die Reaktivierung kann durch das folgende Formelschema veranschaulicht werden:

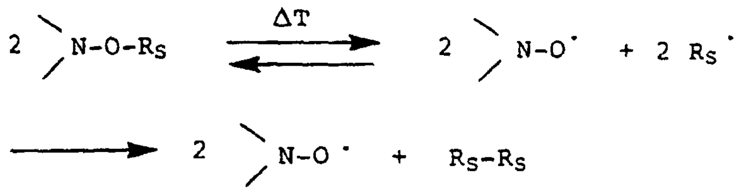


worin $R_S$ für einen einen oder mehrere Styrolreste umfassenden organischen Rest steht. Die Bindung des Restes $R_S$ zum Sauerstoffatom des Nitroxylradikals ist bei erhöhter Temperatur reversibel. Bei Temperaturerhöhung liegt in einer Gleichgewichtsreaktion eine stationäre Konzentration freier $R_S$-Radikale vor, die paarweise kombinieren können, wobei die Nitroxylradikale wieder freigesetzt werden.

Als Maß für die Rückführung von N-Oxyl-Radikalen, die im zurückgeführten Strom der Hochsiederfraktion enthalten sind, läßt sich die Zahl der Zyklen Z definieren, die die N-Oxyl-Radikale die (n-1)-te Destillationskolonne im Durchschnitt durchlaufen. Die Zyklenzahl Z ist über die folgende Gleichung mit dem Anteil x der zurückgeführten Menge Hochsiederfraktion, bezogen auf die Gesamtmenge an Hochsiederfraktion, die im Sumpf der n-ten Destillationskolonne anfällt, verknüpft:

$$Z = \frac{1}{1-x}$$

Vorzugsweise durchlaufen die N-Oxyl-Radikale die (n-1)-te Destillationskolonne im Durchschnitt mindestens 1,4 mal, vorzugsweise 2,0 mal, insbesondere 2,5 mal, besonders bevorzugt 3 mal. Mit den genannten Zyklenzahlen korrespondieren im Allgemeinen Anteile von mehr als 0,3, vorzugsweise mehr als 0,5, insbesondere mehr als 0,6, besonders bevorzugt mehr als 0,67, der zurückgeführten Stabilisatorlösung. Im Allgemeinen ist die Rückführung von 10 bis 90 Gew.-%, vorzugsweise 30 bis 85 Gew.-%, insbesondere 50 bis 80 Gew.-%, der im Sumpf der n-ten Destillationskolonne anfallenden Hochsiederfraktion bevorzugt.

Es wurde gefunden, dass eine besonders gute Reaktivierung der zurückgeführten Stabilisatorlösung erreicht werden kann, wenn der Teilstrom vor der Rückführung auf eine Temperatur von mehr als 130°C erwärmt wird. In einer bevorzugten Ausführungsform des erfindungsgemäßen Verfahrens wird der Teilstrom der Lösung des Stabilisatorsystems vor der Rückführung daher auf eine Temperatur von mehr als 130°C, insbesondere 135-160°C, erwärmt. Das Erwärmen kann zweckmäßigerweise über eine Dauer von 1 bis 300 min, vorzugsweise 10 bis 60 min, durchgeführt werden.

Gemäß einer weiteren bevorzugten Ausführungsform des erfindungsgemäßen Verfahrens wird der Teilstrom der Lösung des Stabilisatorsystems vor der Rückführung mit Sauerstoff behandelt. Die Behandlung mit Sauerstoff kann bei einer Temperatur von 20 bis 200°C, vorzugsweise 50 bis 170°C und insbesondere 100 bis 150°C, vorgenommen werden. Die Behandlung mit Sauerstoff kann mit Vorteil mit einem sauerstoffhaltigen Gasgemisch vorgenommen werden, insbesondere einem im wesentlichen aus Sauerstoff und Stickstoff bestehendem Gasgemisch, wobei dessen Sauerstoffgehalt 3 bis 10 Vol.-% beträgt. Ein geeignetes sauerstoffhaltiges Gasgemisch ist z.B. sogenannte Magerluft. Die Behandlung kann bei Normaldruck oder überatmosphärischem Druck erfolgen. Die Behandlung mit Sauerstoff führt zu einer effektiven Regenerierung freier N-Oxyl-Radikale.

Zur Durchführung der Sauerstoffbehandlung sind alle Vorrichtungen geeignet, die das Inkontaktbringen einer Flüssigkeit, insbesondere einer viskosen Flüssigkeit, mit einem Gas gestatten, z.B. Vorrichtungen zum Durchperlen einer Flüssigkeit mit Gas, zum Einpressen eines Gasstroms in einen Flüssigkeitsstrom usw. Es können auch geeignete Mischbehälter, z.B. gerührte Mischbehälter, vorgesehen werden.

In einer bevorzugten Ausführungsform des erfindungsgemäßen Verfahrens enthält das Stabilisatorsystem ferner wenigstens einen Polymerisationsverzögerer. Als Polymerisationsverzögerer werden Substanzen bezeichnet, die eine radikalisch initiierte Polymerisation der Styrolmonomeren nicht vollständig unterbinden, sondern die Polymerisationsgeschwindigkeit herabsetzen. Die Kombination der erfindungsgemäß zu verwendenden N-Oxyl-Radikale mit wenigstens einem Polymerisationsverzögerer weist den Vorteil auf, dass, etwa im Falle einer Betriebsstörung, wenn die Konzentration an N-Oxyl-Radikalen unterhalb eines für eine wirksame Inhibierung erforderlichen Schwellenwert sinkt, keine schlagartig einsetzende Polymerisation der im System befindlichen Monomerenmenge erfolgt.

Vielmehr findet ein langsamer Anstieg des Oligomer- bzw. Polymergehalts statt, so dass erforderlichenfalls Gegenmaßnahmen ergriffen werden können. Die Kombination der N-Oxyl-Radikale mit einem Polymerisationsverzögerer weist ferner einen synergistischen Effekt auf. Dieser ist darin zu sehen, dass sich die unterschiedlichen Wirkungsmechanismen ergänzen, wobei bei gleicher Gesamtkonzentration des Stabilisatorsystems bei Verwendung einer Kombination von N-Oxyl-Radikalen mit einem Polymerisationsverzögerer eine höhere polymerisationsinhibierende

Wirkung erreicht wird als bei isolierter Verwendung von N-Oxyl-Radikalen oder Polymerisationsverzögerern. Vorzugsweise wird der Polymerisationsverzögerer in einer Menge von 50 bis 2000 ppm, bezogen auf Styrol, eingesetzt. Das Gewichtsverhältnis von N-Oxyl-Radikalen zu Polymerisationsverzögerer liegt vorzugsweise in einem Bereich von 1: 20 bis 20:1.

Als Polymerisationsverzögerer sind insbesondere aromatische Nitroverbindungen, insbesondere der Formel III geeignet

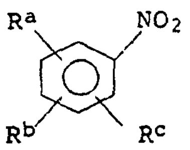 (III),

worin

$R^a$, $R^b$ und $R^c$ unabhängig voneinander jeweils Wasserstoff, $C_1$-$C_6$-Alkyl, Halogen oder einen Rest der Formel CN, SCN, NCO, OH, $NO_2$, COOH, CHO, $SO_2H$ oder $SO_3H$ bedeuten,

wobei der aromatische Ring benzoanelliert sein kann.

In Frage kommende Verbindungen sind beispielsweise

1,3-Dinitrobenzol, 1,4-Dinitrobenzol, 2,6-Dinitro-4-methylphenol, 2-Nitro-4-methylphenol, 2,4,6-Trinitrophenol, 2,4-Dinitro-1-naphthol, 2,4-Dinitro-6-methylphenol, 2,4-Dinitrochlorbenzol, 2,4-Dinitrophenol, 2,4-Dinitro-6-sec-butylphenol, 4-Cyano-2-nitrophenol oder 3-Iod-4-cyano-5-nitrophenol. Bevorzugt werden aromatische Nitroverbindungen, wie 2,6-Dinitro-4-methylphenol, 2-Nitro-4-methylphenol, 2,4-Dinitro-6-sec-butylphenol bzw. 2,4-Dinitro-6-methylphenol verwendet.

Das Stabilisatorsystem im erfindungsgemäßen verfahren kann gegebenenfalls noch einen oder mehrere Costabilisatoren aus der Gruppe der aromatischen Nitrosoverbindungen, Phenothiazine, Chinone, Hydrochinone und deren Ether, Phenole und deren Ether, Hydroxylamine und Phenylendiamine, enthalten.

Weitere Costabilisatoren können auch substituierte Phenole oder Hydrochinone, beispielsweise die folgenden:

4-tert-Butylbrenzcatechin, Methoxyhydrochinon,
2,6-Di-tert-butyl-4-methylphenol,
n-Octadecyl-β-(3,5-di-tert-butyl-4-hydroxyphenyl)-propionat,
1,1,3-Tris-(2-methyl-4-hydroxy-5-tert-butylphenyl)-butan,
1,3,5-Trimethyl-2,4,6-tris-(3,5-di-tert-butyl-4-hydroxybenzyl)-benzol,
1,3,5-Tris-(3,5-di-tert-butyl-4-hydroxybenzyl)-isocyanurat,
1,3,5-Tris-[β-(3,5-di-tert-butyl-4-hydroxyphenyl)-propionyl- oxyethyl-isocyanurat,
1,3,5-Tris-(2,6-dimethyl-3-hydroxy-4-tert-butylbenzyl)-isocyanurat oder
Pentaerythrit-tetrakis-[β-(2,5-di-tert-butyl-4-hydroxy-phenyl)-propionat] sein.

In einer bevorzugten Ausführungsform des erfindungsgemäßen Verfahrens enthält das Stabilisatorsystem neben den erfindungsgemäß verwendeten N-Oxyl-Radikalen ferner einen Aktivator. Als Aktivator wird eine chemische Verbindung bezeichnet, die die Wirkung der N-Oxyl-Radikale steigern können, indem sie z.B. Radikalkombinationsreaktionen katalysieren.

Vorzugsweise wird der Aktivator in einer Menge von 0,01 bis 20 Gew.-%, bezogen auf die N-Oxyl-Radikale, eingesetzt.

Als Aktivatoren sind insbesondere Eisenverbindungen oder andere Übergangsmetallverbindungen geeignet, insbesondere solche, die in verschiedenen wertigkeitsstufen vorliegen können.

Bevorzugte als Aktivatoren geeignete Eisenverbindungen sind ausgewählt aus der Gruppe der

a) Eisencarbonyle und Carbonylferrate,
b) metallorganischen Eisencarbonylverbindungen,
c) unsubstituierten und substituierten Ferrocen-Verbindungen,
d) Eisenverbindungen mit Liganden, welche als Donoratome alleine oder in Mischung Sauerstoff, Stickstoff, Schwefel oder Phosphor enthalten,
e) Eisenhalogenid- und Eisenpseudohalogenidverbindungen.

Unter die Gruppe a) fallen beispielsweise Verbindungen wie Eisenpentacarbonyl, $Fe(CO)_5$, Dieisennonacarbonyl, $Fe_2(CO)_9$, Trieisendodecacarbonyl, $Fe_3(CO)_{12}$, oder Hexaeisenoctadecacarbonyl, $Fe_6(CO)_{18}$, welche durchweg in wenig polaren oder unpolaren Medien löslich sind. weiter sind hier zu nennen die Carbonylferrate wie

$M_2Fe(CO)_4$, $M_2Fe_2(CO)_8$ und $M_2Fe_3(CO)_{11}$, wobei M für ein Äquivalent eines Alkali- oder Erdalkalimetalls steht. Vorzugsweise werden die entsprechenden Na-Verbindungen eingesetzt.

Metallorganische Eisencarbonylverbindungen der Gruppe b) sind beispielsweise Verbindungen der Formel

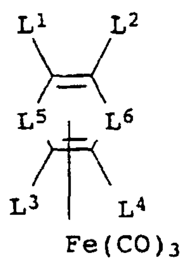

oder

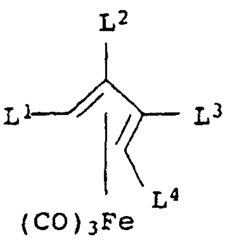

wobei die Variablen bedeuten

$L^1$ - $L^4$ Wasserstoff, $C_1$-$C_4$-Alkyle wie Methyl, Ethyl, Propyl oder t-Butyl

$L^5$, $L^6$ $-(CH_2)_n-$ oder -CO-, wobei für die Variablen $L^5$ und $L^6$ n unabhängig voneinander 0, 1, 2 oder 3 bedeutet.

Geeignete Verbindungen sind z. B.

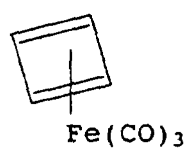

,

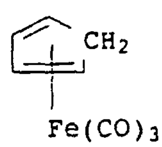

,

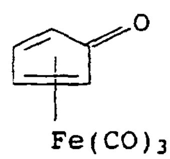

Zu den erfindungsgemäß einzusetzenden Verbindungen der Gruppe c) zählen das Ferrocen selbst sowie die an einem oder beiden Cyclopentadienylringen substituierten Derivate. Weiter können auch dimere Ferrocenderivate eingesetzt werden.

Als Verbindungen der Gruppe d) können beispielsweise eingesetzt werden Komplexe oder Salze des Fe(II) /Fe(III) mit 0-haltigen Liganden wie Sulfat, Acetat, Oxalat, Citrat, Tartrat, Lactat, Gluconat oder Acetylacetonat (acac).

weitere ausschließlich oder überwiegend O-haltige Liganden für Fe(II) oder Fe(III) können aber auch mehrfache cyclische Ether wie Sphäranden, Cryptanden, Cryptasphäranden, Hemisphäranden, Coronanden oder offenkettige Vertreter dieser Ether sowie Podanden sein.

Weiter können verwendet werden Komplexe mit N-haltigen Chelat-Liganden wie Ethylendiamin (en), 1,10-Phenanthrolin (phen), 1,8-Naphthpyridin (napy), 2,2'-Bipyridin (pipy) und Dibenzo[b,i]-1,4,8,11-tetraaza-(14)annulen (taa), aber auch Komplexe des Eisens mit verschiedenen, substituierten Porphyrinliganden, wie sie aus der Literatur bekannt sind (beispielsweise B. Mennier, Chem. Rev., Vol 92 (8), S. 1411-1456, 1992). Andere N-haltige Liganden sind das Phthalocyanin und Derivate davon.

Mit N,O-haltigen Liganden, wie Ethylendiamintetraessigsäure (EDTA) oder Nitrilotriessigsäure (NTA), ergeben sich Verbindungen wie

$[Fe(EDTA)(H_2O)]^{\ominus/2\ominus}$, $[Fe(NTA)(H_2O)_2]$ bzw. $[Fe(NTA)(H_2O)_2]^{\ominus}$,

mit 8-Hydroxychinolin (chin) oder 5-Methyl-8-hydroxychinolin ($H_3$Cchin) Verbindungen wie

$[Fe(chin)_3]/[Fe(chin)_3]^{2\ominus}$ bzw.

$[Fe(H_3C\text{-}chin)_3]/[Fe(H_3C\text{-}chin)_3]^{2\ominus}$,

welche sich ebenfalls verwenden lassen.

Weitere erfindungsgemäß einzusetzende Fe-Verbindungen sind Fe-Komplexe mit Schiff-Basen von Salicylaldehyden.

Die Herstellung dieser N,O-haltigen Liganden ist bekannt und erfolgt in der Regel durch Kondensation von aromatischen oder heteroaromatischen $\alpha$-Hydroxyaldehyden mit einem aliphatischen oder aromatischen Diamin oder Mehrfachamin. Anschließend erfolgt die Umsetzung der Liganden mit einem Fe-Salz in wäßriger Lösung.

Andere verwendbare Fe-Verbindungen mit S-haltigen Liganden sind etwa

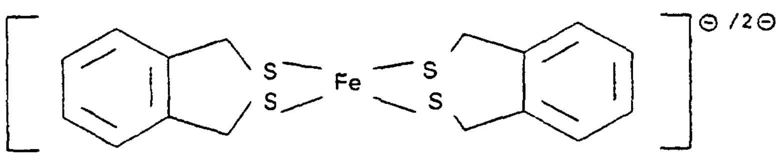

oder $[Fe_4S_4(SR)_4]^{4\ominus/3\ominus}$, aber auch Komplexe des Fe(II)/Fe(III) mit

Dithiocarbonaten $R_2NCS_2^{\ominus}$ wie etwa $[Fe(S_2CNR_2)_3]^{\ominus}$ (R=$CH_3$, $C_2H_5$).

Weiter lassen sich auch Verbindungen der Gruppe e) einsetzen. Bevorzugt werden bei den Fe-Halogeniden die Fe(II)- und Fe(III)-Salze von Cl und Br, sowie die Komplexverbindungen $FeX_4^{\ominus/2\ominus}$ (X=Cl, Br) eingesetzt. Zu den erfindungsgemäß einzusetzenden Fe-Pseudohalogenid-Verbindungen zählen beispielsweise $[Fe(CN)_6]^{3\ominus}/[Fe(CN)_6]^{4\ominus}$ sowie Thiocyanatkomplexe der Reihe $[Fe(SCN)_{3-X}(H_2O)_{3+X}]^{X\oplus}$ (x = 0, 1, 2).

Als Gegenionen aller aufgeführten negativ geladenen Komplexionen finden bevorzugt $H^{\oplus}$, $Na^{\oplus}$, $K^{\oplus}$ und Ammoniumionen $NH_4^{\oplus}$ sowie $N(CH_3)_4^{\oplus}$, bei den Hexacyanoferraten aber neben $K^{\oplus}$ auch $Fe^{2\oplus}$ im

Falle des $[Fe(CN)_6]^{3\ominus}$ und $Fe^{3\oplus}$ im Falle des $[Fe(CN_6]^{4\ominus}$ Einsatz.

Bei den aufgeführten positiv geladenen Komplexionen werden bevorzugt als Gegenionen $Cl^{\ominus}$, $Br^{\ominus}$, $I^{\ominus}$, $SO_4^{2\ominus}$, $H_3CCO_2^{\ominus}$, $CrO_4^{2\ominus}$, $BF_4^{\ominus}$ sowie $B(C_6H_5)_4^{\ominus}$ eingesetzt.

Die Erfindung wird nun durch das nachfolgende Beispiel näher veranschaulicht.

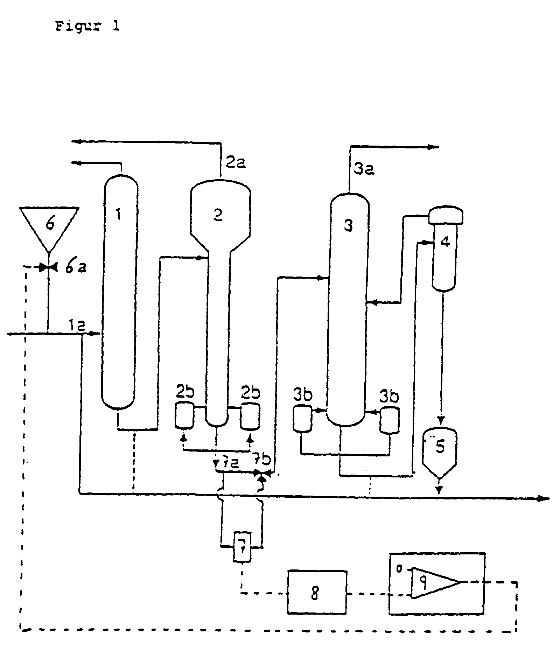

In einer Destillationsanlage gemäß Fig. 1 wird kontinuierlich Rohstyrol destilliert, das aus der Dehydrierung von Ethylbenzol stammt.

Die verwendete Destillationsanlage besteht aus einer sog. Benzol-(Toluol-)kolonne 1, der ein Gemisch aus z.B. im Wesentlichen Styrol, Ethylbenzol, Benzol und Toluol 1a zugeführt wird, einer sogenannten Ethylbenzolkolonne 2, die der Abtrennung und Rückgewinnung des Ethylbenzols 2a dient und der sogenannten Styrolkolonne 3, aus der schließlich das Reinstyrol 3a gewonnen wird.

Ethylbenzolkolonne 2 und Styrolkolonne 3 sind jeweils mit Aufkochern 2b bzw. 3b versehen, d.h. sie besitzen einen beheizbaren Sumpf.

Der Sumpfaustrag der Kolonne 3 wird der im Wesentlichen aus den Apparaten 4 und 5 bestehenden Einrichtung zugeführt. Dabei ist 4 ein als Dünnfilmverdampfer oder Flashverdampfer ausgebildeter Konzentrator, in dem der aus dem Kolonnensumpf 3 entnommene Produktstrom von Leichtsiedern befreit wird. Die Leichtsieder werden in einer Aufarbeitungskolonne (nicht dargestellt) nochmals in Styrol und $\alpha$-($\beta$-)Methylstyrol aufgetrennt. Ein Teilstrom des aus 4 erhaltenen und in 5 zwischengespeicherten Konzentrats wird zum Zulauf der Kolonne 1 und/oder 2 zurückgeführt.

Über den T-Verteiler 7a und das Umlenkventil 7b wird periodisch Sumpfaustrag aus der Ethylbenzolkolonne 2 durch die integrierte ESR-Messzelle 7 geleitet (Magnet und wechselfeldquelle sind nicht dargestellt). Das in der Messzelle 7 detektierte Signal wird über einen Signalumformer 8 einem Komparator 9 mit wählbarem Vorgabewert zugeführt. Der Vorgabewert ist so gewählt, dass er einer N-Oxyl-Radikalkonzentration von etwa 200 ppm, bezogen auf Rohstyrol entspricht. Der Komparator 9 steuert die Zugabe einer Lösung des Stabilisatorsystems, die im Vorratsbehälter 6 gelagert wird, über das Dosierventil 6a. Das verwendete Stabilisatorsystem enthält 1-Oxyl-2,2,6,6-tetramethylpiperidin-4-ol (Hydroxy-TEMPO).

**Patentansprüche**

1. Verfahren zur Verhinderung unerwünschter Polymerisation in einem ethylenisch ungesättigte Verbindungen enthaltenden Stoffgemisch durch Aufrechterhalten einer wirksamen Konzentration eines Stabilisatorsystems, das N-Oxyl-Radikale umfasst, wobei

   (i) periodisch oder kontinuierlich ein elektronisches Signal gewonnen wird, das mit der Konzentration der N-Oxyl-Radikale in dem Stoffgemisch korreliert,

   (ii) das elektronische Signal mit einem Vorgabewert verglichen wird und

   (iii) nach Maßgabe des Vergleichs ein Zusatz des Stabilisatorsystems zum Stoffgemisch gesteuert wird.

2. Verfahren nach Anspruch 1 zur Verhinderung unerwünschter Polymerisation bei der Gewinnung von ethylenisch ungesättigten Verbindungen aus einem diese enthaltenden Stoffgemisch durch Destillation.

3. Verfahren nach Anspruch 1 oder 2, wobei das elektronische Signal gewonnen wird, indem das Stoffgemisch oder eine Teilmenge davon einem magnetischen Feld ausgesetzt wird und gleichzeitig ein elektromagnetisches Wechselfeld eingestrahlt wird, wobei die durch die N-Oxyl-Radikale hervorgerufene Resonanz detektiert wird.

4. Verfahren nach Anspruch 2 oder 3, bei dem die Destillation in einer Kaskade von mehreren Destillationskolonnen erfolgt, wobei sich im Sumpf mindestens einer Destillationskolonne eine das Stabilisatorsystem enthaltende Hochsiederfraktion anreichert, wobei ein Teilstrom der Hochsiederfraktion entnommen und dem Zulauf einer vorgeschalteten Kolonne beigemischt wird.

5. Verfahren nach einem der Ansprüche 1 bis 4, wobei die Konzentration der N-Oxyl-Radikale in dem Stoffgemisch 5 bis 150 ppm, bezogen auf ethylenisch ungesättigte Verbindung, beträgt.

6. Verfahren nach einem der Ansprüche 1 bis 5, wobei das Stabilisatorsystem ferner einen Polymerisationsverzögerer enthält.

7. Verfahren nach Anspruch 6, wobei es sich bei dem Polymerisationsverzögerer um eine aromatische Nitroverbindung handelt.

8. Verfahren nach einem der Ansprüche 1 bis 7, wobei es sich bei der ethylenisch ungesättigten Verbindung um eine vinylaromatische Verbindung handelt.

9. Verfahren nach einem der vorhergehenden Ansprüche, wobei der Vorgabewert von wenigstens einer weiteren Meßgröße, die unter einer Temperatur, einem Redoxpotential, einer NIR-Transmission oder -Absorption, einer Trübung, einer Viskosität, einer Dichte oder einem Brechungsindex ausgewählt ist, abhängig ist.

**Claims**

1. A process for preventing undesired polymerization in a mixture containing ethylenically unsaturated compounds by maintaining an effective concentration of a stabilizer system which comprises N-oxyl radicals, wherein

   (i) an electronic signal which correlates with the concentration of the N-oxyl radicals in the mixture is obtained periodically or continuously,

   (ii) the electronic signal is compared with a reference value and

   (iii) an addition of stabilizer system to the mixture is controlled according to the comparison.

2. A process as claimed in claim 1 for preventing undesired polymerization during the isolation of ethylenically unsaturated compounds from a mixture containing them by distillation.

**3.** A process as claimed in claim 1 or 2, wherein the electronic signal is obtained by exposing the mixture or a portion thereof to a magnetic field and simultaneously applying an alternating electromagnetic field, the resonance caused by the N-oxyl radicals being detected.

**4.** A process as claimed in claim 2 or 3, in which the distillation is carried out in a cascade of a plurality of distillation columns, wherein a high boiler fraction containing the stabilizer system accumulates in the bottom of at least one distillation column and a part-stream of the high boiler fraction is removed and is mixed with the feed of an upstream column.

**5.** A process as claimed in any of claims 1 to 4, wherein the concentration of the N-oxyl radicals in the mixture is from 5 to 150 ppm, based on ethylenically unsaturated compound.

**6.** A process as claimed in any of claims 1 to 5, wherein the stabilizer system furthermore contains a polymerization retardant.

**7.** A process as claimed in claim 6, wherein the polymerization retardant is an aromatic nitrocompound.

**8.** A process as claimed in any of claims 1 to 7, wherein the ethylenically unsaturated compound is a vinylaromatic compound.

**9.** A process as claimed in any of the preceding claims, wherein the reference value is dependent on at least one further measured variable which is selected from a temperature, a redox potential, an NIR transmission or absorption, a turbidity, viscosity, density or a refractive index.

**Revendications**

**1.** Procédé pour empêcher la polymérisation indésirable dans un mélange de substances contenant des composés insaturés éthyléniques par maintien d'une concentration efficace d'un système stabilisant qui contient des radicaux N-oxyle, dans lequel

(i) un signal électronique qui est en corrélation avec la concentration des radicaux N-oxyle dans le mélange de substances, est périodiquement ou continuellement acquis,
(ii) le signal électronique est comparé avec une valeur donnée et
(iii) si on s'en tient à la comparaison, un supplément du système stabilisant est conduit au mélange de substances.

**2.** Procédé selon la revendication 1 pour empêcher la polymérisation indésirable, dans lequel l'obtention de composés insaturés éthyléniques à partir d'un mélange de substances contenant ceux-ci s'effectue par distillation.

**3.** Procédé selon la revendication 1 ou 2, dans lequel le signal électronique est acquis, en ce que le mélange de substances ou une quantité partielle de celui-ci est soumis à un champ magnétique et simultanément un champ alternatif électromagnétique est rayonné, par lequel la résonance provoquée par les radicaux N-oxyle est détectée.

**4.** Procédé selon la revendication 2 ou 3, dans lequel la distillation s'effectue dans une cascade de plusieurs colonnes de distillation, dans lequel dans le bas d'au moins une colonne de distillation une fraction à point d'ébullition élevé contenant le système stabilisant s'enrichit, dans lequel un courant partiel de la fraction à point d'ébullition élevé est prélevé et est mélangé à l'alimentation d'une colonne disposée en amont.

**5.** Procédé selon l'une des revendications 1 à 4, dans lequel la concentration en radicaux N-oxyle dans le mélange de substances représente 5 à 150 ppm par rapport au composé insaturé éthylénique.

**6.** Procédé selon l'une des revendications 1 à 5, dans lequel le système stabilisant comprend en outre un retardateur de polymérisation.

**7.** Procédé selon la revendication 6, dans lequel il s'agit en tant que retardateur de polymérisation d'un composé

nitré aromatique.

8. Procédé selon l'une des revendications 1 à 7, dans lequel il s'agit en tant que composé insaturé éthylénique d'un composé vinyle aromatique.

9. Procédé selon l'une des revendications précédentes, dans lequel la valeur donnée est dépendante d'au moins une grandeur de mesure supplémentaire, qui est choisie parmi une température, un potentiel redox, une transmission ou une absorption NIR, une turbidité, une viscosité, une densité ou un indice de réfraction.

Figur 1